# EUROPEAN PATENT APPLICATION

(11) **EP 3 409 206 A1**
(43) Date of publication of application: **05.12.2018**
(21) Application number: 17173527.7
(22) Date of filing: 30.05.2017
(51) Int. Cl.: A61B 6/00

(54) **MOBILE C-ARM X-RAY IMAGING SYSTEM**

(71) Applicant: Koninklijke Philips N.V., 5656 AE Eindhoven (NL)
(72) Inventor: Mungase, Ganesh, 5656 AE Eindhoven (NL)
(74) Representative: de Haan, Poul Erik

(57) **Abstract**

The present invention relates to mobile C-arm X-ray imaging. In order to provide an improved and facilitated positioning of a mobile C-arm system, a mobile C-arm X-ray imaging system (10) is provided comprising a C-arm imaging assembly (12) with a C-arm device (14), and a mobile stand assembly (18) comprising a first base (20), and a second base (22). The C-arm imaging assembly is coupled to the mobile stand assembly allowing a horizontal pivoting movement of the C-arm device. One of the first base and second base is pivotable in a horizontal plane.

## Description

### FIELD OF THE INVENTION

The present invention relates to a mobile C-arm X-ray imaging system and a corresponding method for adjusting a position of a mobile C-arm imaging device.

### BACKGROUND OF THE INVENTION

Mobile C-arm X-ray imaging systems are used, for example, during invasive surgical procedure, examination procedures and the like. Mobile C-arm X-ray imaging systems can be relatively bulky and correct positioning may be difficult. Positioning of the C-arm in relation to the patient may necessitate rotating the entire mobile X-ray system. US 2012 0219122 A1 relates to a wheeled C-arm support device for a mobile C-arm X-ray machine. The C-arm support device includes two steerable front wheels, two freely movable rear wheels and a steering unit. However, it has been shown that due to increasing demands for a precise positioning, the positioning of a C-arm of mobile X-ray systems can be cumbersome.

### SUMMARY OF THE INVENTION

There may thus be a need to provide an improved and facilitated positioning of a mobile C-arm system.

The object of the present invention is solved by the subject-matter of the independent claims; further embodiments are incorporated in the dependent claims. It should be noted that the following described aspects of the invention apply also for the mobile C-arm X-ray imaging system and for the method for adjusting a position of a mobile C-arm imaging device.

According to a first aspect of the invention, a mobile C-arm X-ray imaging system is provided comprising a C-arm imaging assembly with a C-arm device. Further, the C-arm imaging assembly comprises a mobile stand assembly comprising a first base and a second base. The C-arm assembly is coupled to the mobile stand assembly allowing a horizontal pivoting movement of the C-arm device. One of the first and second base is pivotable in a horizontal plane.

In an example, the first base is coupled to the second base such that the first base follows the horizontal pivoting movement of the C-arm device.

The term "horizontal" (or "vertical") relates to a normal way of operation of the imaging system when the mobile system is arranged on a horizontal floor plane.

The term "horizontal pivoting movement" refers to pivoting movement about an axis perpendicular to the floor plane, and can also be referred to as swiveling movement.

In an example, the C-arm device is coupled to the mobile stand assembly via a C-arm bearing or support arrangement allowing a horizontal pivoting movement.

In an example, the first base is coupled to the second base via a base bearing or support arrangement.

In an example, the second base is temporarily stationary. The first base is pivotable in relation to the second base when following the horizontal pivoting movement of the C-arm device.

The pivotable base of the first and second base can be referred to as the pivotable base.

The first base can be arranged as the base closer to a space where a patient support like a patient table is arrangeable for the imaging. The second base would then be the base away from that patient space.

The first base could then be referred to as "front base", and the second base could then be referred to as "rear base".

In one example, the first base, e.g. the front base, is pivotable and the second base, e.g. the rear base, remains temporarily fixed.

In another example, the second base, e.g. the rear base, is pivotable and the first base, e.g. the front base, remains temporarily fixed.

In an example, the pivoting base is assigned to provide a support to the ground floor for the C-arm in spatial relation to the horizontally moved, i.e. pivoted C-arm. The pivoting base follows the C-arm in the same rotation direction in relation to the vertical pivoting axis. The other, so-to-speak non-pivoting base provides and acts as a counterweight. As an advantage, the base to be moved is the lighter base of the two.

In another example, the base assigned to provide a support to the ground floor for the C-arm is the so-to-speak non-pivoting base. The other, pivoting base provides and acts as a counterweight that follows the movement of the C-arm. As an advantage, the counterweight is arranged with maximum lever arrangement in relation to the C-arm. The pivoting base follows the C-arm in the same rotation direction in relation to the vertical pivoting axis.

The C-arm device can also be adapted to be moved in a number of further degrees of freedom, such that the C-arm device can be arranged in relation to the operation table in the most suitable manner for the present situation and imaging task, for example determined by a surgeon.

In an example, a further bearing, such as a sleeve-like bearing structure, could be arranged at the C-arm device allowing rotation of the C-arm device in a vertical plane.

As indicated above, as a result, in one example, the stability of the C-arm is maintained by pivoting the first part of the base along with the C-arm assembly. The C-arm is stable for at least 90 degrees' movement as the first base follows the movement of the C-arm. The 0°-degree position can be defined as being the position when the C-arm is positioned over the operation table. In other words, the movement of the C-arm spans an angle of 180 degrees. In another example, the C-arm movement provides a full range movement flexibility and spans an angle of 360 degrees. The first base following the C-arm ensures the stability of the system, as the weight of the C-arm assembly gets balanced during a full range of pivoting.

This provides additional imaging flexibility and a unique usability pattern of positioning, in addition to the conventional C-arm features and capabilities.

In an example, the first base can be designed with a reduced extension or length. This reduces the possibility of a collision with other equipment such as the patient table and thus ensures an improved reach of the C-arm suitable for different clinical procedures. As a result, the mobile C-arm X-ray imaging can be designed in a much more compact manner and space efficient manner. This is of high importance, as operation theatres may lack space availability for the staff.

According to an example, the pivotable base of the first and second base is pivotably connected to the other one of the first and second base.

According to an example, the mobile stand assembly can partly be temporarily fixed in a position in relation to a ground floor such that the pivotable base of the first and second base can be pivoted whereas the other one of the first and second base remains fixed.

Hence, a pivotable base and a non-pivotable base are provided, wherein the pivoting-function relates to a surrounding structure, since the pivoting between two base portions is a movement relative to each other.

In an example, the C-arm device is connected to the first base, such that the first base follows the horizontal pivoting movement of the C-arm device.

In an example, the connection can be provided as mechanical coupling. In a further option, the connection is a data connection, e.g. the movement is supported by motor-drives. In a further example, the first base comprises a drive actuator, which is electronically coupled to the pivoting movement of the C-arm device.

According to an example, the C-arm device is cantilevering from the mobile stand assembly in a cantilevering direction, and, in the cantilevering direction, the first base is arranged in front of the second base.

In a first option i), the first base is the pivotable base, and the C-arm device is coupled to the first base such that the first base follows the horizontal pivoting movement of the C-arm device.

For example, the first base follows the horizontal pivoting movement of the C-arm device to be arranged at least partly below the C-arm device during the horizontal pivoting movement of the C-arm device.

In a second option ii), the second base is the pivotable base and the C-arm device is coupled to the second base such that the second base follows the horizontal pivoting movement of the C-arm device.

For example, the second base follows the horizontal pivoting movement of the C-arm device to be arranged at least partly opposite balancing point provided by ground support means to achieve an effective counterweight levering distance if the second base acting as counterweight for the cantilevering C-arm during the horizontal pivoting movement of the C-arm device.

According to an example, a ground support of the pivotable base is also movable along a radial direction during the horizontal pivoting movement of the C-arm device.

The C-arm support arrangement can also be referred to as C-arm bearing arrangement.

The horizontal pivoting movement of the C-arm refers to the pivoting movement of the C-arm device in the horizontal plane.

The base support arrangement can also be referred to as base bearing arrangement.

According to a second aspect of the invention, there is provided a method for adjusting a position of a mobile C-arm imaging device, comprising the following steps. In a first step, also referred to as step a), a C-arm device of a C-arm imaging assembly moves in relation to a mobile stand assembly of the C-arm imaging assembly in a first horizontal pivoting movement. In a second step, also referred to as step b), a first base of the mobile stand assembly moves in a second horizontal pivoting movement. The first base is coupled to the second base such that the first base follows a horizontal pivoting movement of the C-arm device.

In an example, step b) is provided in a synchronous manner.

In an example, the C-arm is moved and adjusted to different settings based on the procedure. For example, the C-arm is moved horizontally, vertically and around a swivel axis, so that X-ray images of the patient can be acquired from almost any angle.

According to an aspect of the invention, a base of a mobile stand assembly of an C-arm X-ray imaging system is split into a first part and a second part. The first base is coupled to the C-arm device and follows its horizontal pivoting movement. The first base ensures the stability of the system as weight of the C-arm assembly gets balanced during horizontal pivoting movement, which enables to increase the range of pivoting and, as another effect, to reduce the footprint and increase the space use efficiency of the mobile C-arm X-ray imaging device.

The compact C-arm can be used in confined spaces, providing extra space for staff and other equipment. The C-arm provides additional imaging flexibility and unique usability pattern of positioning. The C-arm enables efficient workflow by reducing the time consumed in various C-arm movements during the procedures. The C-arm provides quick and ease of positioning with minimal operator movements. The invention provides best position for the operator and avoids all the movements including moving back and forth and around the system several times to adjust the position of the C-arm as per the surgeon's requirement. The C-arm enables all controls for C-arm positioning within operator's immediate reach. The unique usability pattern of positioning allows C-arm adjustment, controls and user console or interface by standing in one position without any additional movement and allows use of C-arm with minimal interference to the staff and the equipment in the operating room. Further, it provides full visibility to the operator for C-arm positioning over the area of interest. The improved visibility will reduce the time required for positioning the C-arm. The full range of swivel eliminates the need for removal of entire C-arm systems away from operating region to make free space for surgeon. The operating region can be cleared by swivel movement only instead of moving entire system. The recall of swivel movement is much easier than recall of entire system to the original clinical position. The swivel is possible in smaller operating rooms also to keep the C-arm away from the operating region and bring back as and when required. Further, the image display visibility for the surgeon will not be hampered in some of the clinical procedures as the distance of the display is shorter due to the shorter first base.

These and other aspects of the present invention will become apparent from and elucidated with reference to the embodiments described hereinafter.

### BRIEF DESCRIPTION OF THE DRAWINGS

Exemplary embodiments of the invention will be described in the following with reference to the following drawings.
Fig. 1 illustrates an example of a mobile C-arm X-ray imaging system in a side view.
Fig. 2 illustrates a cross view of the mobile stand assembly of the mobile C-arm X-ray imaging.
Fig. 3 illustrates two exemplary positions of a mobile C-arm X-ray imaging system in a top view.
Fig. 4 illustrates a corresponding method of a mobile C-arm X-ray imaging system.

### DETAILED DESCRIPTION OF EMBODIMENTS

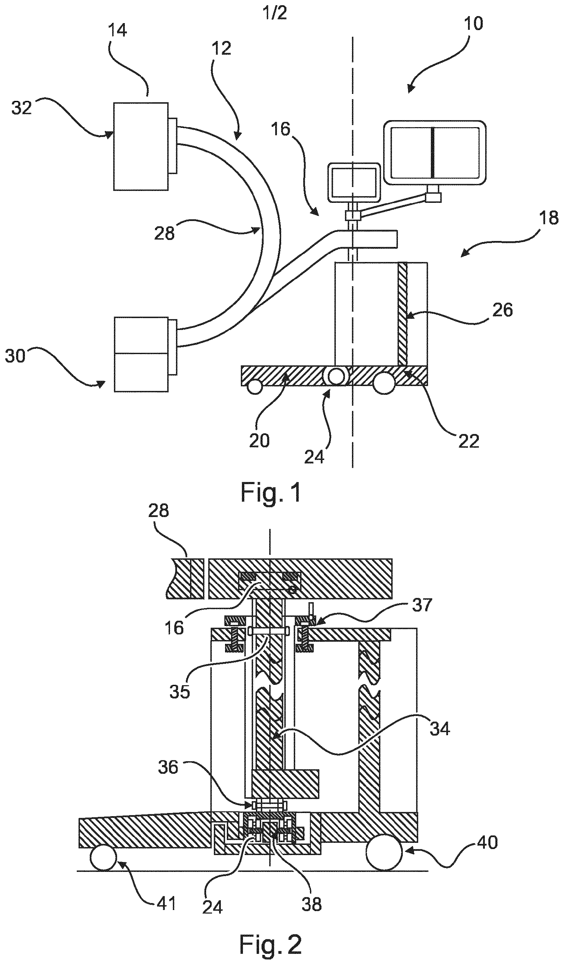

Fig. 1 illustrates a mobile C-arm X-ray imaging system 10 comprising a C-arm imaging assembly 12 with a C-arm device 14. Further, the C-arm imaging assembly comprises a mobile stand assembly 18 comprising a first base 20 and a second base 22. The C-arm assembly is coupled to the mobile stand assembly allowing a horizontal pivoting movement of the C-arm device. One of the first and second base is pivotable in a horizontal plane.

In an example, one of the first and second base is coupled to the second base such that the first base follows the horizontal pivoting movement of the C-arm device.

In an example, the C-arm device is coupled to the mobile stand assembly via a C-arm bearing arrangement 16 allowing a horizontal pivoting movement.

In an example, the first base 20 is coupled to the second base 22 via a base support (or bearing) arrangement 24.

In an example, the second base is temporarily stationary. The first base is pivotable in relation to the second base when following the horizontal pivoting movement of the C-arm device.

As an example, the pivotable base of the first and second base is pivotably connected to the other one of the first and second base.

As a further example, the mobile stand assembly can partly be temporarily fixed in a position in relation to a ground floor such that the pivotable base of the first and second base can be pivoted, whereas the other one of the first and second base remains fixed.

The first base and second base form a supporting structure of the mobile stand assembly. The supporting structure allows the cantilevering of the C-arm imaging assembly. As the imaging system is mobile, e.g. provided with wheels or other moving structure, the supporting structure provides secure hold of the C-arm assembly. The supporting structure thus acts as the base for the C-arm. In an example, a supporting frame 26 is arranged in the second base and is dimensioned in weight such that the C-arm assembly is further balanced by a counter weight.

In an example, the C-arm device is cantilevering from the mobile stand assembly in a cantilevering direction, and, in the cantilevering direction, the first base is arranged in front of the second base. In a first example, the first base is the pivotable base, and the C-arm device is coupled to the first base such that the first base follows the horizontal pivoting movement of the C-arm device. In a second example, the second base is the pivotable base and the C-arm device is coupled to the second base such that the second base follows the horizontal pivoting movement of the C-arm device.

The C-arm device can also be referred to as C-arm device. The C-arm device comprises a C-arm 28 as the structural part, an X-ray source 30 and an X-ray detector 32 as the functional parts mounted to the C-arm. The X-ray detector can be an image intensifier or a flat panel detector.

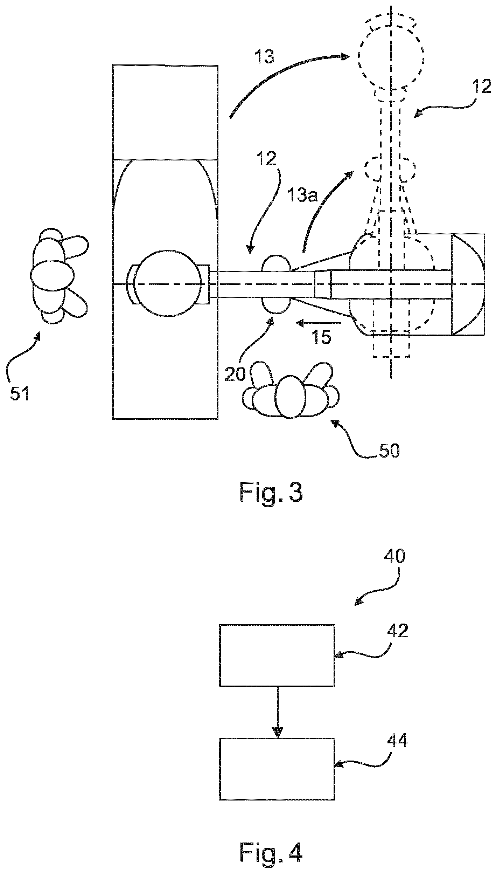

In an example, the C-arm device is connected to the first base, such that the first base follows the horizontal pivoting movement of the C-arm device as can be seen in Fig. 3.

In an example, the connection can be provided as a mechanical coupling. In a further option, the connection is a data connection, e.g. when the movement is supported by motor-drives. In a further example, the first base comprises a drive actuator, which is electronically coupled to the pivoting movement of the C-arm device.

Fig. 2 illustrates an example of a cross view of the mobile stand assembly of the mobile C-arm X-ray imaging.

In an example, the mobile C-arm further comprises a first vertical shaft 34. The C-arm device is connected to a C-arm support arrangement, or bearing arrangement 16 that is coupled to a top of the first vertical shaft 35 and a bottom 36 of the first vertical shaft is coupled to a base support arrangement 24 connected to the pivotable base to transmit the horizontal pivoting movement of the C-arm device to the pivotable base.

In an example (not shown), both ends of the first shaft are secured by using fasteners.

In an example, the pivoting of the C-arm device can also be transmitted without the first shaft. In an example the transmission of the pivoting movement of the C-arm device to the first base is transmitted by a cable or rope arrangement.

Further, as shown in Fig. 2 the mobile stand assembly further comprises a second shaft 38. In an example (not shown), the base support arrangement 24 comprises an inner ring and an outer ring. The inner ring is mounted on the second shaft 38. The outer ring holds the pivotable base and the bottom of the first shaft transmitting the pivoting movement of the C-arm device to the pivotable base.

In an example, the second shaft can be any other element to connect the inner ring to the mobile stand assembly.

According to another example (not shown), the C-arm bearing arrangement comprises a swivel brake assembly such that the C-arm device can be locked to a required angle. The locking of the C-arm device to a required angle can be aligned preferably with the locking of the first base. In an example, the swivel brake assembly comprises a brake connection line and a brake lever to lock the c-arm assembly at a required angle.

In an example (not shown), the mobile stand assembly further comprises hardware components, an operating panel, a housing and handles attached to the housing. The hardware components comprise at least one of the group of a generator, a transformer, control units, workstations, power supply, and cables. The operating panel can be a user interface. The housing may comprise aesthetic covers. In an example, the operating panel is connected to the housing by a supporting structure allowing the operating panels to pivot freely.

In an example, the second base comprises a wheel arrangement 40. In an example, the wheel arrangement comprises two wheels, which are lockable by a stand brake (not shown). In an example, the wheels are steerable.

In a further example, the first base comprises a wheel arrangement 41 with at least one wheel. The wheel arrangement 41 is configured such that a rolling direction of the at least one wheel follows a pre-defined curve that is concentric with the horizontal pivoting movement of the pivotable base.

In an example, the first base contains one wheel. In another example, two or more wheels are provided. In an example, the two wheels are arranged in a manner that a predetermined radius is set-up for the rolling direction.

In an example (not shown), the first shaft 34 is height adjustable. In a further example (not shown), the first shaft comprises an actuator for height adjusting.

Fig. 3 shows the different positions of the mobile C-arm imaging system and an operator 50 and a surgeon 51 interacting with the mobile C-arm X-ray imaging system. The continuous line illustrates mobile C-arm imaging assembly 12 in a first position. In this position, the C-arm imaging assembly is positioned at the operating table to support the surgeon. The operator can assist the surgeon in positioning the C-arm assembly. The dashed line, shows the C-arm imaging assembly 12 a in a second position. It can also be referred to as a parking position. The parking position is arranged besides the table to not provide an obstacle for the surgeon for further treatment steps.

As an example, Fig. 3 shows a movement from the first position to the second position (shown by the arrow 13), the C-arm imaging assembly is pivoted by -90 degrees into the second position. However, other positions of the C-arm imaging assembly are possible. The C-arm imaging assembly can be moved in the full range of 360 degrees. The first base 20 following the horizontal pivoting movement of the C-arm imaging assembly is further illustrated (shown by arrow 13a). In an example, the horizontal pivoting movement spans an angle from at least 45 degrees, preferably at least 90 degrees, and more preferably 0 to 180 degrees, and more preferably 0 to 360 degrees.

Fig. 3 further show the operator arranged next to the mobile device, since the following of the first base part to the C-arm device allows improved handling of the mobile C-arm imaging device. The surgeon and the operator discuss the procedure and best positioning of a C-arm. The operator follows the instructions and tries to position the C-arm in an optimal way. Thus, the system enables all controls for the C-arm positioning within the operators' sight and reach. Further, the first base of the mobile C-arm imaging device does not hit the patient table and restrict the C-arm reach to the patient in some of the procedures anymore, since it can be arranged with shorter length as the flowing pivoting movement improves stability.

Thus, the mobile C-arm imaging system provides quick and easy positioning with minimal operator movements. Thus, best position for the operator is provided. The unique usability pattern of positioning allows C-arm adjustment, controls and user console or interface by standing in one position without any additional movement. The need for removal of entire C-arm systems away from operating region to make free space for surgeon is avoided. The full range of pivoting eliminates the need for a removal of the entire C-arm system away from operating region. The operating region can be cleared by the swivel movement only instead of moving entire system. Further, the recall of a swivel movement is much easier than the recall of the entire system movement to facilitate the original clinical position. The swiveling is possible in smaller operation theatres. The C-arm can be kept away from the operating region and brought back when required without arranging the full C-arm system again, i.e. without moving the full base. In an example, the first base is arranged below the suspension arrangement such that the first base is arranged within the vertical plane of the suspension arrangement.

According to an example, the first base is also movable along a radial direction during the horizontal pivoting movement of the C-arm device (shown by arrow 15, 15a). For example, the first base is extendable.

The radial direction can also be referred to as a direction aligned to the cantilever direction of the C-arm or as a direction along the direction of the C-arm device can be referred to as a movement of the first base along a connection line or a suspension between the C-arm device and the mobile stand assembly. In other words, the first base follows the horizontal pivoting movement, while moving along the connection line at the same time. In an example, the first base can be extended to achieve a better cantilevering relation of the (heavy) C-arm in relation to the ground support, and thus to minimize the cantilevering distance.

In an example, the first base can be extended to increase the distance of the ground support in relation to the second base.

In an example, the first base can be moved towards the C-arm device to improve stability for determined angular deflection (to be described further in the figures). In an example, the movement along a direction of the C-arm device can be controlled electronically and/or mechanically (e.g. ropes, hydraulics, etc.).

In an example, the horizontal pivoting movement spans an angle from 0 to 180 degrees, preferably from 0 to 360 degrees.

Fig. 4 illustrates a corresponding method 40 for adjusting a position of a mobile C-arm imaging device. The method comprises the following steps. In a first step 42, also referred to as step a), a C-arm device of a C-arm imaging assembly moves in relation to a mobile stand assembly of the C-arm imaging assembly in a first horizontal pivoting movement. In an example, the C-arm imaging assembly is coupled to the mobile stand assembly comprising a first base and a second base, from which one is pivotable in a horizontal plane. In a second step 44, also referred to as step b), a first base of the mobile stand assembly moves in a second horizontal pivoting movement.

In an example, the first base is coupled to the second base such that the first base follows a horizontal pivoting movement of the C-arm device.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

## Claims

1. A mobile C-arm X-ray imaging system (10) comprising:
- a C-arm imaging assembly (12) with a C-arm device (14); and
- a mobile stand assembly (18) comprising:
- a first base (20); and
- a second base (22);
wherein the C-arm imaging assembly is coupled to the mobile stand assembly allowing a horizontal pivoting movement of the C-arm device; and wherein one of the first base and second base is pivotable in a horizontal plane.

2. Mobile C-arm X-ray imaging system according to claim 1, wherein the pivotable base of the first and second base is pivotably connected to the other one of the first and second base.

3. Mobile C-arm X-ray imaging system according to claim 1 or 2, wherein the mobile stand assembly can partly be temporarily fixed in a position in relation to a ground floor such that the pivotable base of the first and second base can be pivoted, whereas the other one of the first and second base remains fixed.

4. Mobile C-arm X-ray imaging system according to one of the preceding claims, wherein the C-arm device is cantilevering from the mobile stand assembly in a cantilevering direction, and wherein, in the cantilevering direction, the first base being arranged in front of the second base; and wherein
i) the first base is the pivotable base, and the C-arm device is coupled to the first base such that the first base follows the horizontal pivoting movement of the C-arm device; or
ii) the second base is the pivotable base and the C-arm device is coupled to the second base such that the second base follows the horizontal pivoting movement of the C-arm device.

5. Mobile C-arm X-ray imaging system according to one of the preceding claims, wherein the mobile stand assembly further comprises a first vertical shaft (34); and
wherein the C-arm device is connected to a C-arm support arrangement (16) that is coupled to a top of the first vertical shaft (35), and a bottom (36) of the first vertical shaft is coupled to a base support arrangement (24) connected to the pivotable base to transmit the horizontal pivoting movement of the C-arm device to the pivotable base.

6. Mobile C-arm X-ray imaging system according to one of the preceding claims, wherein a ground support of the pivotable base is also movable along a radial direction during the horizontal pivoting movement of the C-arm device.

7. Mobile C-arm X-ray imaging system according to one of the preceding claims, wherein the mobile stand assembly further comprises a second shaft (38);
wherein the base support arrangement comprises an inner ring and an outer ring;
wherein the inner ring is mounted on the second shaft; and
wherein the outer ring holds the pivotable base and the bottom of the first shaft transmitting the pivoting movement of the C-arm device to the pivotable base.

8. Mobile C-arm X-ray imaging system according to one of the preceding claims, wherein the C-arm bearing arrangement comprises a swivel brake assembly such that the C-arm device can be locked to a required angle; and
wherein, preferably, the locking of the C-arm device to a required angle can be aligned with the locking of the pivotable base.

9. Mobile C-arm X-ray imaging system according to one of the preceding claims, wherein the pivotable base comprises a wheel arrangement (41) with at least one wheel; and
wherein the wheel arrangement is configured such that a rolling direction of the at least one wheel follows a pre-defined curve that is concentric with the horizontal pivoting movement of the pivotable base.

10. Mobile C-arm X-ray imaging system according to one of the preceding claims, wherein the horizontal pivoting movement spans an angle from at least 45 degrees, preferably at least 90 degrees, and more preferably 0 to 180 degrees, and more preferably 0 to 360 degrees.

11. A method (40) for adjusting a position of a mobile C-arm imaging device, comprising the following steps:
a) moving (42) a C-arm device of a C-arm imaging assembly in relation to a mobile stand assembly of the C-arm imaging assembly in a first horizontal pivoting movement; wherein the C-arm imaging assembly is coupled to the mobile stand assembly comprising a first base and a second base, from which one is pivotable in a horizontal plane;
b) moving (44) the pivotable base in a second horizontal pivoting movement.
